**Europäisches Patentamt**

**European Patent Office**

**Office européen des brevets**

(11) Publication number: **0 138 687**
**B1**

# EUROPEAN PATENT SPECIFICATION

(45) Date of publication of patent specification: **08.07.87**

(51) Int. Cl.⁴: **A 61 B 17/08, A 61 B 17/10**

(21) Application number: **84401936.4**

(22) Date of filing: **27.09.84**

(54) Surgical fastener exhibiting improved hemostasis.

(30) Priority: **04.10.83 US 538930**

(43) Date of publication of application:
**24.04.85 Bulletin 85/17**

(45) Publication of the grant of the patent:
**08.07.87 Bulletin 87/28**

(84) Designated Contracting States:
**AT BE CH FR IT LI NL SE**

(56) References cited:
**EP-A-0 077 262**
**GB-A- 972 731**

(73) Proprietor: **United States Surgical Corporation**
**150 Glover Avenue**
**Norwalk Connecticut 06850 (US)**

(72) Inventor: **Korthoff, Herbert W.**
**110 Old Belden Hill Road**
**Wilton Connecticut 06897 (US)**
Inventor: **Green, David T.**
**251 Wolfpit Road**
**Norwalk Connecticut 06851 (US)**

(74) Representative: **Martin, Jean-Jacques et al**
**Cabinet REGIMBEAU 26, Avenue Kléber**
**F-75116 Paris (FR)**

Courier Press, Leamington Spa, England.

## Description

### Background of the Invention

This invention relates to surgical fasteners (e.g., staples), and more particularly to a fastener comprising a fastener member and a retainer member and exhibiting improved hemostasis.

Surgical fastening devices allow a surgeon to fasten body tissue by applying surgical fasteners. The fasteners may be applied singly in succession or a number may be applied simultaneously. Surgical fasteners are often made of metals such as tantalum and stainless steel, which are inert. Fasteners of magnesium, which fasteners are gradually absorbed by the body, are also known.

Non-metallic fasteners are also known and may be preferable to metal fasteners in some procedures. For example, non-metallic fasteners can be made X-ray transparent so that they do not scatter X-rays and therefore do not degrade the quality of radiographs as may happen when metal fasteners are used.

On the other hand, objects of non-metallic resinous materials are usually too resilient (i.e., elastic) to hold deformed shapes (assuming plastic flow does not occur). (As used herein, the term "resinous materials" means non-metallic materials, such as natural or synethetic polymers and resins, including protein-based materials, which are relatively flexible and elastic, and which may or may not be absorbable into the body). The greater elasticity of resinous materials generally makes it impossible to directly substitute such materials for the metal in conventional surgical fasteners.

To overcome this problem, resinous surgical fasteners may be made of two parts: a fastener member and a retainer member. The legs or prongs of the fastener member are driven through one side of the tissue to be fastened and the retainer member interlocks with the prongs of the fastener member on the other side of the tissue to hold the entire fastener structure in place. One such fastener structure and apparatus for applying it are disclosed in Green U.S. patent 4,402,445, issued September 6, 1983, which is hereby incorporated by reference in its entirety.

A frequent goal in fastening tissue is achieving hemostasis along the fastener line. Hemostasis is achieved by exerting pressure on the tissue from both sides. If metal staples are used, that pressure (hereinafter referred to as "hemostatic pressure") is exerted by and between the base of staple on one side of the tissue and the crimped legs on the other side of the tissue. In typical crimped metal staples no part of of the staple extends beyond the ends of the base. Therefore, a second staple can be applied very close to the first staple, so that the bases of the two staples are in a line. In that case the gap between staples can be quite small so that hemostatic pressure is applied uniformly along the entire staple line.

In contrast, when two-part resinous fasteners are used, hemostatic pressure is exerted by and between the retainer member and the base of the fastener member. In known two-part resinous fasteners the prongs of the fastener member extend from the ends of the base. The retainer member is typically longer than the distance between the prongs and therefore must extend beyond the fastener member base. Accordingly, the bases of adjacent fastener members lying in line are separated by at least the sum of the distances by which adjacent retainer members extend beyond the associated fastener member bases. Thus, there are gaps between adjacent fastener members. Full hemostatic pressure is not exerted on the tissue in these gaps.

One way to make up for the above-mentioned gaps in a line of resinous fasteners is to apply the fasteners in two parallel rows, with a linear offset between the rows so that the gaps in one row are opposite the bases of the fastener members of the other row. However, this doubles the number of fasteners that are required and increases the area of tissue affected by the fasteners.

Another typical characteristic of resinous materials is that they are not as strong as metals. Surgical fasteners of resinous materials may therefore tend to deform during application to tissue. In particular, the fastener member prongs may tend to splay or spread apart as the prongs are forced through the tissue. One way to overcome this tendency is to provide a metal guide pin adjacent each prong to help the prong penetrate the tissue without deformation. After the fasteners have been applied, the guide pins are withdrawn from the tissue. This guide pin structure has the disadvantage that it increases the complexity and cost of the apparatus for applying the fasteners.

### Summary of the Invention

In accordance with this invention, a surgical fastener that exerts hemostasic presure long its entire length and cooperates with the applying apparatus to minimize splaying of the prongs is provided. The surgical fastener comprises a fastener member and a retainer member and is applied to tissue in a row in which the lengths of the fastener are in a line and in which adjacent fasteners are nearly touching each other.

The fastener member comprises (1) a base and (2) a pair of substantially parallel prongs extending substantially perpendicularly from the base in substantially the same direction, each prong being spaced inward from its respective end of the base. Preferably, the base is the same length as the retainer member. Also preferably, the prongs are substantially equidistant from the transverse centerline of the base.

A fastener-applying apparatus for use with the fastener of this invention contains a number of pusher members corresponding to the number of fasteners to be fired. A plurality of retainer members and a corresponding number of fastener members are positioned within the apparatus. The pusher members force the fastener members through tissue into engagement with the retainer member by pushing the bases of the fastener members. The length of each pusher member parallel to the longitudinal axis of the associated fastener member base is substantially equal to the length of the associated fastener member base, and each pusher member exerts pressure along substantially the entire length of each fastener member base.

The present invention finds its greatest use with surgical fasteners of resinous materials, although the invention may be used with fasteners of other materials that have separate fastener and retainer members.

Thus, the present invention provides a surgical fastener that exerts hemostatic pressure along its entire length and cooperates with fastener-applying apparatus to reduce splaying of the prongs of the fastener while the fastener is being applied to tissue.

Brief Description of the Drawings

The above and other advantages of the present invention will be more apparent after consideration of the accompanying drawings in which like parts are indicated by like reference characters throughout and in which:

FIG. 1 is an elevational view of a known surgical fastener;

FIG. 2 is a cross-sectional view of tissue fastened with such fasteners;

FIG. 3 is a fragmentary plan view of tissue fastened with such fasteners;

FIG. 4 is an elevational view of a surgical fastener according to the present invention;

FIG. 5 is a cross-sectional view of tissue fastened with such fasteners; and

FIGS. 6 and 7 are, respectively, fragmentary cross-sectional views of the fastener members of FIGS. 1 and 4 being applied to tissue by the pusher members of fastener-applying apparatus.

Detailed Description of the Invention

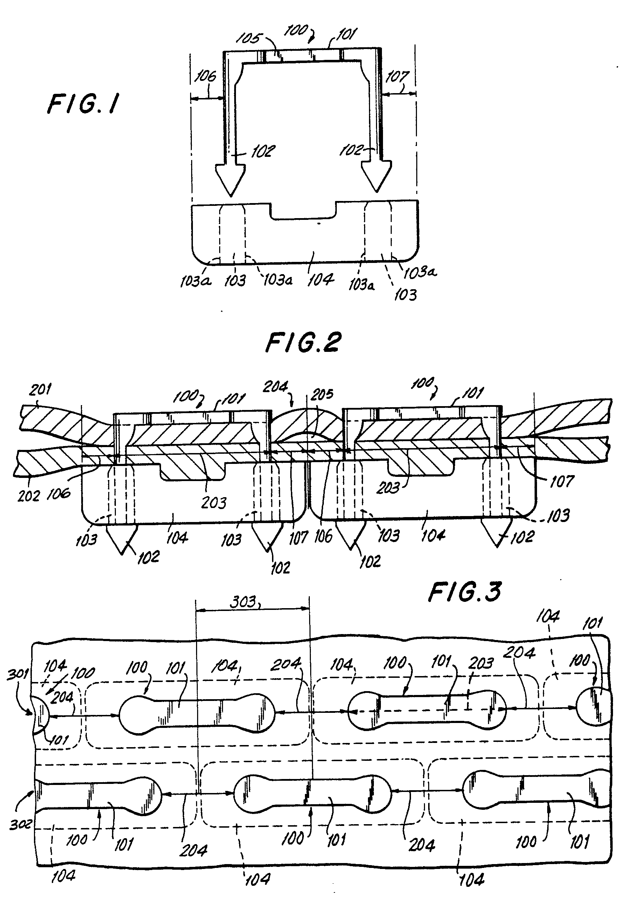

Surgical fastener 100 in Fig. 1 includes retainer member 104 and fastener member 101, which has two prongs 102 depending from base 105. Prongs 102 are driven through tissue to engage apertures 103, whose sidewalls are indicated by dashed lines 103a, in retainer member 104. Both members may be made from a resinous material which may or may not be absorbable in the body.

When fastener 100 is applied to tissue, full hemostatic pressure may not be exerted in the areas represented by arrows 106 and 107. This can be seen more clearly in FIG. 2, which shows two fasteners 100 applied to tissue layers 201 and 202. Full hemostatic pressure is exerted along area 203, but such pressure may not be exerted in area 204, which includes adjacent areas 106 and 107. As a result, there is a gap 205 through which body fluids (e.g., blood, plasma) may seep.

Thus, when using fasteners 100, two offset rows 301 and 302 of fasteners are customarily used (see FIG. 3). Because of offset 303 between rows 301 and 302, each area 204 in one row is opposite an area 203 in the other row. Seepage of fluids through any gaps 205 in one row is arrested at corresponding areas 203 in the other row.

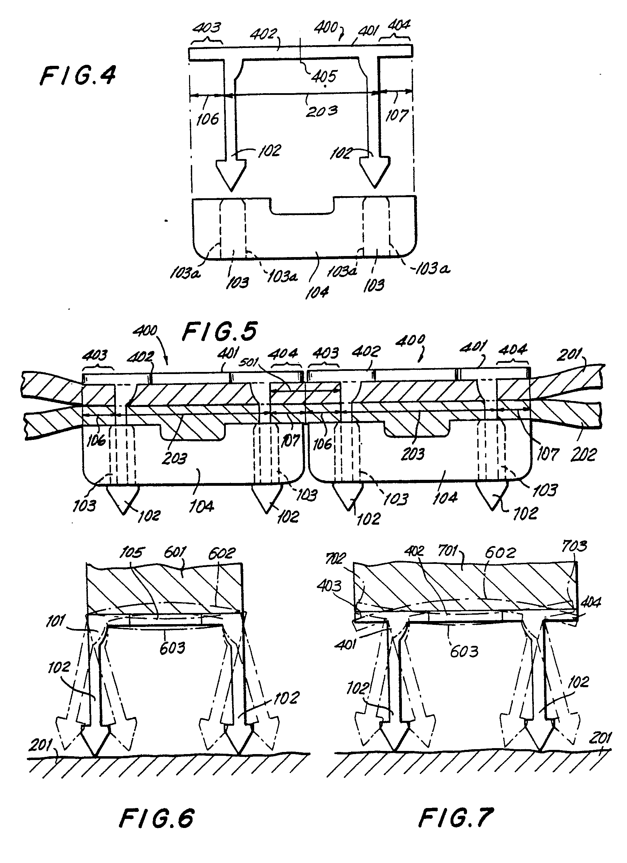

The present invention reduces or eliminates the need for a second row of fasteners to provide total hemostasis. Fastener 400 according to the present invention is shown in FIG. 4. Preferred fastener member 401 can be described as "pi-shaped" bacause of its resemblance to the Greek letter pi. Base 402 of fastener member 401 has extensions 403 and 404, which extend over areas 106 and 107. The retainer member and the base of the fastener member have substantially the same length; the sum of the lengths of extensions 403 and 404 is substantially equal to the sum of the lengths of areas 106 and 107 of FIG. 1. Thus, hemostatic pressure is exerted between extensions 403 and 404 and the corresponding portions of retainer member 104 extending beyond each aperture 103. By applying fasteners 400 close together in a line, total hemostasis can be provided with only a single row of fasteners.

This is illustrated in FIG. 5. There full hemostatic pressure is exerted in area 501 as well as in areas 203. That is in contrast to the previously known fasteners, as seen in FIG. 2, where full hemostatic pressure may not be exerted in area 204.

Returning to FIG. 4, apertures 103 are usually located symmetrically along the length of retainer member 104 so that areas 106 and 107 are substantially equal in length. Extensions 403 and 404 of base 402 of fastener member 401 are therefore preferably also substantially equal in length, with prongs 102 substantially equidistant from transverse centerline 405 of base 402. However, these conditions are not necessary. It is only necessary that the lengths of the fastener and retainer members be substantially the same. That will provide the desired total hemostasis.

The fasteners shown in FIG. 5 are shown with small gaps between adjacent fasteners. These gaps are shown because it is usually necessary to have some structure in the fastener-applying apparatus (not shown) to separate the channels through which the individual fastener members are guided towards the tissue. During application of the fasteners the tissue is generally compressed by the fastener-applying apparatus, thereby stretching the tissue. When the fastener-applying apparatus is removed from the tissue, the tissue tends to resume its former shape. That brings the fastener in the tissue closer together so that they are touching or nearly touching. It is desirable that adjacent fasteners in the tissue be touching or nearly touching to provide full hemostasis, although there should preferably be some gap because it is also desirable that the fastener line be somewhat flexible on the tissue. (As used herein, "nearly touching" means separated by a gap of between 0 mm and about 0.5 mm).

FIGS. 6 and 7 show the beneficial effect of extensions 403 and 404, in combination with fastener-applying pushers 701 (which are longer, parallel to the longitudinal axis of the fastener member base, than would otherwise be employed), in counteracting the tendency of prongs 102 to splay. FIG. 6 shows known fastener member 101 as it is about to pierce tissue 201 under the downward urging of pusher 601 in known fastener-applying apparatus (not otherwise shown). FIG. 7 shows fastener member 401 of this invention in the same position. In accordance with the present invention, pusher 701 is longer, in the direction parallel to the base of the fastener member, than pusher member 601 for fasteners of comparable size. In particular, pusher member 701 is preferably substantially equal in length in the above-identified direction to base 402 of fastener member 401, including extensions 403 and 404. As a consequence, pusher member 701 contacts fastener member 401 along the entire length of base 402. Pusher member 701 should not be measurably longer in that direction than base 402; otherwise, it would prevent adjacent fastener members from being applied to the tissue as close together as possible, which is necessary to provide hemostasis. In other respects the fastener-applying apparatus may be conventional, as exemplified by the above-identified Green patent, although the present invention may facilitate the provision of fastener-applying apparatus as shown in that patent without the metal guide pins.

During the application of resinous fasteners, because both the tissue and fastener member are non-rigid, prongs 102 may tend to bend inward or outward. If prongs 102 try to bend inward, the base of either fastener member 101 or 401 bows upward as indicated at 602 in FIGS. 6 and 7. Such upward bowing is prevented by the presence of pusher 601 or pusher 701 in either device.

If prongs 102 splay or spread apart, the base of either fastener member 101 or 401 bows downward. A shown in FIG. 6, with known fastener member 101 base 105 is not prevented from bowing downward as indicated at 603. In contrast, for fastener member 401 of this invention, if base 402 tries to bow downward, extensions 403 and 404 try to bend upward, as shown in FIG. 7 at 702 and 703. However, upward movement of extensions 403 and 404 is resisted by pusher 701, which is as long as base 402 and exerts pressure along the entire length of base 402. Thus, the combination of longer pusher 701 and extensions 403 and 404 reduces the tendency of prongs 102 of the new fastener to splay.

**Claims**

1. A surgical fastener (400) for application to body tissue, comprising a fastener member (401) and a retainer member (104), the fastener member (401) comprising a base (402) and a pair of substantially parallel tissue-piercing prongs (102) extending substantially perpendicularly from the base in substantially the same direction, wherein each tissue-piercing prong (102) is spaced inward from its respective end of the base (402) thereby defining tissue-engaging extensions (403, 404) of the base between each prong and its respective end of the base, the length of the base (402) being substantially equal to the length of the retainer member (104), so that hemostatic pressure can be exerted between said extensions and the corresponding portions of retainer member (104).

2. The fastener of claim 1 wherein the fastener member (401) and the retainer member (104) are made of a resinous material.

3. The fastener of claim 2 wherein the resinous material is absorbable in the body.

4. The fastener of claim 1 wherein the tissue-piercing prongs (102) are substantially equidistant from the transverse centreline of the base (402).

5. A row of surgical fasteners (400) for application to body tissue, each of said fasteners comprising a fastener member (401) and a retainer member (104), each fastener member (401) comprising a base (402) and a pair of substantially parallel tissue-piercing prongs (102) extending substantially perpendicularly from the base (402) in substantially the same direction, wherein each tissue-piercing prong (102) of each fastener member is spaced inward from its respective end of the base (402) thereby defining tissue-engaging extensions (403, 404) of the base between each prong and its respective end of the base, the length of the base (402) being substantially equal to the length of the retainer member (104), and the tissue-engaging extensions of each fastener in the row touching or nearly touching a tissue-engaging extension of each fastener adjacent thereto, thereby providing essentially total hemostasis when the fasteners are applied to tissue.

6. In combination:

apparatus for applying a plurality of surgical fasteners to body tissue, each of the fasteners (400) having a fastener member (401) and a retainer member (104);

a plurality of retainer members (104) positioned within the apparatus; and

a corresponding number of fastener members (401) positioned within the apparatus, each of the fastener members (401) comprising a base (402) and a pair of substantially parallel tissue-piercing prongs (102) extending substantially perpendicularly from the base, each tissue-piercing prong (102) being spaced inward from its respective end of the base (402) thereby defining tissue-engaging extensions (403, 404) of the base between each prong and its respective end of the base, the length of the base (402) being substantially equal to the length of the retainer member (104), so that hemostatic pressure can be exerted between said extensions and the corresponding portions of retainer member (104), wherein the apparatus further comprises a number of pusher members (701) corresponding to the number of fastener members (401) for pushing the tissue-piercing prongs of said fastener members through tissue into engagement with the retainer members (104), the length of each pusher member (701) being substantially equal to the length of the base (402) of each fastener member so that when each pusher member contacts the base of its respective fastener member, the pusher member (701) exerts pressure along substantially the entire length of the base (402) of its respective fastener member (401).

7. The combination of claim 7 wherein the fastener members (401) and retainer members (104) are made of a resinous material.

8. The combination of claim 8 wherein the resinous material is absorbable in the body.

9. The combination of claim 7 wherein the tissue-piercing prongs (102) of the fastener member (401) are substantially equidistant from the transverse centreline of the base (402) of the fastener member.

**Patentansprüche**

1. Chirurgisches im Körpergewebe einzusetzendes Befestigungselement (400) mit einem Festlegeteil (401) und einem Halteteil (104), wobei der Festlegeteil (401) einen Steg (402) und zwei im wesentlichen parallele durch das Gewebe durchtretenden Zinken (102) besitzt, die sich im wesentlichen in derselben Richtung im wesentlichen rechwinklig zu dem Steg von diesem weg erstrekken, jede durch das Gewebe durchtretende Zinke (102) im Abstand einwärts von dem benachbarten Ende des Steges (402) angeordnet ist, wobei das Gewebe berührenden Fortsätze (403, 404) des Steges zwischen jeder Zinke und dem benachbarten Ende des Steges gebildet sind, und der Steg (402) im wesentlichen dieselbe Länge hat wie der Halteteil (104), so dass hämostatischer Druck zwischen diesen Fortsätzen und den entsprechenden Teilen des Halteteils (104) ausgeübt werden kann.

2. Befestigungselement nach Anspruch 1, dadurch gekennzeichnet, dass der Festlegeteil (401) und der Halteteil (104) aus einem harzartigen Werkstoff bestehen.

3. Befestigungselement nach Anspruch 2, dadurch gekennzeichnet, dass der harzartige Werkstoff vom Körper absorbierbar ist.

4. Befestigungselement nach Anspruch 1, dadurch gekennzeichnet, dass der durch das Gewebe durchtretenden Zinken (102) im wesentlichen gleichweit von der Quermittellinie des Steges (402) entfernt sind.

5. In einer Reihe in Körpergewebe eingesetzte, chirurgische Befestigungselemente, die je einen Festlegeteil (401) und einen Halteteil (104) besitzen, wobei jeder der Festlegeteile (401) einen Steg (402) und zwei im wesentlichen in derselben Richtung im wesentlichen rechtwinklig zu dem Steg (402) von diesem weg erstreckenden durch das Gewebe durchtretenden Zinken (102) jede durch das Gewebe durchtretende Zinke (102) im Abstand einwärts von dem benachbarten Ende des Steges angeordnet ist, wobei das Gewebe berührenden Fortsätze (403, 404) des Steges zwischen jeder Zinke und dem benachbarten Ende des Steges gebildet sind, und der Steg (402) im wesentlichen dieselbe Länge hat wie der Halteteil (104), und die das Gewebe berührenden Fortsätze jedes Befestigungselement in der Reihe eines das Gewebe berührenden Fortsatz jedes im benachbarten Befestigungselement berührt oder fast berührt, so dass eine im wesentlichen vollständige Hämostase erzielt wird, wenn die Befestigungselemente in das Gewebe eingesetzt sind.

6. In Kombination:
Ein Instrument zum Einsetzen von chirurgischen Befestigungselementen in Körpergewebe, wobei jedes der Befestigungselemente (400) einen Festlegeteil (401) und einen Halteteil (104) besitzt;
mehrere in dem Instrument angeordnete Halteteile (104) und in entsprechender Anzahl in dem Instrument angeordnete Festlegeteile (401), wobei jeder der Festlegeteile (401) einen Steg (402) und zwei im wesentlichen in derselben Richtung im wesentlichen rechtwinklig zu dem Steg von diesem weg erstrekenden durch das Gewebe durchtretenden Zinken (102) besitzt, jede durch das Gewebe durchtretende Zinke (102) im Abstand einwärts von dem benachbarten Ende des Steges (402) angeordnet its, wobei das Gewebe berührenden Fortsätze (403, 404) des Steges zwischen jeder Zinke und dem benachbarten Ende des Steges gebildet sind, und der Steg (402) im wesentlichen dieselbe Länge hat wie der Halteteil (104), so dass hämostatischer Druck zwischen diesen Fortsätzen und den entsprechenden Teilen des Halteteils (104) ausgeübt werden kann, wobei das Instrument in einer der Anzahl der Festlegeteile (401) entsprechenden Anzahl Stössel (701) ferner besitzt, die dazu dienen, die das Gewebe durchtretenden Zinken dieser Festlegeteile durch das Gewebe hindurch bis zum Angriff an den Halteteilen (104) zu schieben, wobei jeder Stössel (701) im wesentlichen ebensolang ist wide der Steg (402) jedes Festlegeteils, so dass jeder Stössel (701) durch den Angriff an dem Steg des zugeordneten Festlegeteils (401) auf dessen Steg einen Druck im wesentlichen auf der ganzen Länge des Steges (402) ausübt.

7. Kombination nach Anspruch 6, dadurch gekennzeichnet, dass die Festlegeteile (401) und Halteteile (104) aus harzartigem Werkstoff bestehen.

8. Kombination nach Anspuch 7, dadurch gekennzeichnet, dass der harzartige Werkstoff vom Körper absorbierbar ist.

9. Kombination nach Anspruch 6, dadurch gekennzeichnet, dass die das Gewebe durchtretenden Zinken (102) des Festlegeteils (401) im wesentlichen gleich weit von der Quermittellinie des Steges (402) des Festlegeteils entfernt sind.

**Revendications**

1. Agrafe chirurgicale (400) pour pose sur des tissus du corps, comportant un élément d'agrafage (401) et un élément de retenue (104), l'élément d'agrafage (401) comprenant une base (402) et une paire de dents (102) sensiblement parallèles, qui transpercent les tissus et qui s'étendent sensiblement perpendiculairement depuis la base, dans sensiblement la même direction, étant précisé que chaque dent (102) qui transperce le tissu est, vers l'intérieur, à une certaine distance de son extrémité respective de la base (402), définissant ainsi des prolongements (403, 404), qui viennent au contact des tissus, de la base entre chaque dent et son extrémité respective de la base, la longueur de la base (402) étant sensiblement égale à la longueur de l'élément de retenue (104), de sorte qu'une pression hémostatique peut s'exercer entre lesdits prolongements et les portions correspondantes de l'élément de retenue (104).

2. Agrafe selon la revendication 1, dans laquelle l'élément d'agrafage (401) et l'élément de retenue (104) sont fabriqués en un matériau à base de résine.

3. Agrafe selon la revendication 2, dans laquelle le matériau résineaux peut être absorbé dans le corps.

4. Agrafe selon la revendication 1, dans laquelle les dents (102) qui transpercent les tissus sont sensiblement équidistantes de l'axe transversal de la base (402).

5. Rangée d'agrafes chirurgicales (400) pour pose sur des tissus du corps, chacune desdites agrafes comportant un élément d'agrafage (401) et un élément de retenue (104), chaque élément d'agrafage (401) comprenant une base (402) en une paire de dents (102) sensiblement parallèles, qui transpercent les tissus et s'étendent sensiblement perpendiculairement depuis la base (402) sensiblement dans la même direction, étant précisé que chaque dent (102), qui transperce les tissus, de chaque élément d'agrafage est, vers l'intérieur, à une certaine distance de son extrémité de la base (402), définissant ainsi des prolongements (403, 404), qui viennent au contact des tissus, de la base entre chaque dent et son extrémité respective de la base, la longueur de la base (402) étant sensiblement égale à la longueur de, l'élément de retenue (104) et les prolongements, qui viennent au contact des tissus, de chaque agrafe de la rangée venant toucher, ou presque toucher, un prolongement, qui vient au contact des tissus, de chaque agrafe qui lui est voisine, assurant par là une hémostase essentiellement totale lorsque l'on pose les agrafes sur le tissue.

6. En combinaison:
un appareil pour poser un pluralité d'agrafes chirurgicales sur des tissus du corps, chacune des agrafes (400) comportant un élément d'agrafage (401) et un élément de retenue (104);
une pluralité d'éléments de retenue (104) placés à l'intérieur de l'appareil; et
un nombre correspondant d'éléments d'agrafage (401) placés à l'intérieur de l'appareil, chacun des éléments d'agrafage (401) comprenant une base (402) et une paire de dents (102) sensiblement parallèles, qui transperecent les tissus et qui s'étendent sensiblement perpendiculairement depuis la base, chaque dent (102) qui transperce le tissu étant, vers l'intérieur, à une certaine distance de son extrémité de la base (402), définissant ainsi des prolongements (403, 404), qui viennent au contact des tissus, de la base entre chaque dent et son extrémité respective de la base, la longueur de la base (402) étant sensiblement égale à la longueur de l'élément de retenue (104), de sorte qu'une pression hémostatique peut s'exercer entre lesdits prolongements et les portions correspondantes de l'élément de retenue (104), étant précisé que l'appareil comporte en outre un nombre d'éléments poussoirs (701) correspondant au nombre d'éléments d'agrafage (401) pour pousser les dents, qui transpercent les tissus, desdits éléments d'agragage à travers les tissus pour venir en contact avec les éléments de retenue (104), la longeur de chaque élément poussoir (701) étant sensiblement égale à la longueur de la base (402) de chaque élément d'agrafage de sorte que, lorsque chaque élément poussoir vient au contact de la base de son élément d'agrafage respectif, l'élément poussoir (701) exerce une pression le long de, sensiblement, la totalité de la longueur de la base (402) de son élément d'agrafage respectif (401).

7. La combinaison de la revendication 6, dans laquelle les éléments d'agrafage (401) et les éléments de retenue (104) sont fabriqués en un matériau à base de résine.

8. La combinaison de la revendication 7, dans laquelle le matériau à base de résine peut être absorbé dans le corps.

9. La comnbinaison de la revendication 6, dans laquelle les dent (102) de l'élément d'agrafage (401) qui transpercent les tissus sont sensiblement équidistants de l'axe transversal de la base (402) de l'élément d'agrafage.

*FIG.1*

*FIG.2*

*FIG.3*

1

FIG.4

FIG.5

FIG.6

FIG.7